# EUROPEAN PATENT APPLICATION

(11) **EP 4 471 208 A1**
(43) Date of publication of application: **04.12.2024**
(21) Application number: 23763261.7
(22) Date of filing: 16.02.2023
(51) Int. Cl.: D21H 21/16, C09D 7/63, D21H 27/00, D06M 13/35, C09K 3/18

(54) **WATER-/OIL-REPELLENT AGENT**

(30) Priority: 02.03.2022 JP 2022031824
(71) Applicant: DAIKIN INDUSTRIES, LTD., Osaka-shi, Osaka 530-0001 (JP)
(72) Inventor: IKEUCHI, Hideyuki, Osaka-Shi, Osaka 530-0001 (JP); SAKAMAKI, Tatsunori, Osaka-Shi, Osaka 530-0001 (JP)
(74) Representative: Hoffmann Eitle
(86) International application number: PCT/JP2023/005476
(87) International publication number: WO 2023/167002

(57) **Abstract**

Through use of this water-/oil-repellent agent including a modified heterocyclic compound having a heterocycle and a C1-40 hydrocarbon group, adequate oil-resistance can be imparted to a substrate. The heterocycle is preferably a nitrogen-containing heterocycle. The hydrocarbon group is preferably an octyl group, a decyl group, an undecyl group, a lauryl group, a tridecyl group, a myristyl group, a palmityl group, a cetyl group, an isopalmityl group, a stearyl group, or a behenyl group. The heterocycle is preferably pyrimidine, pyridazine, pyridine, diazine, triazine, oxazole, or oxadiazole.

## Description

### Technical Field

The present disclosure relates to a water- and oil-repellent agent using a modified heterocyclic ring compound obtained by modifying a heterocyclic ring compound.

### Background Art

Paper containers are promising as an alternative to disposable plastic containers. Paper-made food packaging materials and food containers are required to prevent oozing-out of moisture and oil content of foods and water- and oil-repellent agents (for example, oil-resistant agents) are applied to papers by internal addition or external addition.
Patent Literature 1 (JP 2013-87371 A) discloses an oilproof paper provided with an oil-resistant layer obtained by applying and drying an oil-resistant layer coating material containing an acrylic resin on at least one surface of a paper support, wherein the oil-resistant layer coating material contains a thickener having, as a main component, at least one alkali-soluble polymer emulsion.
Patent Literature 2 (JP 2006-342110 A) discloses a method of producing a triazine compound to improve the thermal recording material printability.
Patent Literature 3 (WO2009/148703) discloses use of a triazine compound as a charge additive to change charges of an electret article.

### Citation List

### Patent Literature

Patent Literature 1: JP 2013-87371 A
Patent Literature 2: JP 2006-342110 A
Patent Literature 3: WO 2009/148703

### Summary of Invention

### Technical Problem

An object of the present disclosure is to provide a water- and oil-repellent agent which can impart sufficient oil-resistance and water-repellency and/or oil-repellency.

### Solution to Problem

The present disclosure relates to a modified heterocyclic ring compound of which raw material heterocyclic ring compound is so modified as to have an organic modifying group. An example of the organic modifying group is a hydrocarbon group having 1 to 40 carbon atoms and optionally having a substituent.

The present disclosure provides a water dispersion composition containing the modified heterocyclic ring compound having a hydrocarbon group having 1 to 40 carbon atoms and a heterocyclic ring, and a surfactant.

The modified heterocyclic ring compound (water dispersion composition) can be used as a water- and oil-repellent agent, for example, an oil-resistant agent.

Preferable aspects of the present disclosure are as follows.
Aspect 1: A water- and oil-repellent agent, comprising a modified heterocyclic ring compound having a hydrocarbon group having 1 to 40 carbon atoms and a heterocyclic ring.
Aspect 2: The water- and oil-repellent agent according to Aspect 1, wherein the heterocyclic ring is a nitrogen-containing heterocyclic ring.
Aspect 3: The water- and oil-repellent agent according to Aspect 1 or 2, wherein the hydrocarbon group is at least one selected from an octyl group, a decyl group, an undecyl group, a lauryl group, a tridecyl group, a myristyl group, a palmityl group, a cetyl group, an isopalmityl group, a stearyl group and a behenyl group.
Aspect 4: The water- and oil-repellent agent according to any one of Aspects 1 to 3, wherein the heterocyclic ring is at least one selected from the group consisting of pyrimidine, pyridazine, pyridine, diazine, triazine, oxazole, oxadiazole, isoxazole, imidazole and pyrazine.
Aspect 5: The water- and oil-repellent agent according to any one of Aspects 1 to 4, wherein the modified heterocyclic ring compound is a compound represented by the formula:

   A(-B-R)ₙ

   wherein
   A is an optionally substituted heterocyclic ring;
   B is -NR²-, -O-, -C(=O)NR²-, -NR²C(=O)-, -C(=O)O-, -OC(=O)-, -NR²C(=O)NR²-, or -OC(=O)NR²-, wherein R² are identical or different and each a hydrogen atom or a hydrocarbon group having 1 to 10 carbon atoms;
   R is a hydrocarbon group having 1 to 40 carbon atoms and optionally having a substituent; and
   n is a number of 1 to 5.
Aspect 6: The water- and oil-repellent agent according to any one of Aspects 1 to 5, wherein the water- and oil-repellent agent exhibits a contact angle of n-hexadecane of 10° or larger.
Aspect 7: The water- and oil-repellent agent according to any one of Aspects 1 to 6, wherein the modified heterocyclic ring compound has a melting point of 40°C or higher or exhibits no melting point.
Aspect 8: The water- and oil-repellent agent according to any one of Aspects 1 to 7, wherein the water- and oil-repellent agent in a solution concentration of 14.8 mg/mL has a viscosity of 5 cP or higher and 100 cP or lower.
Aspect 9: The water- and oil-repellent agent according to any one of Aspects 1 to 8, wherein the water- and oil-repellent agent is a water dispersion composition.
Aspect 10: The water- and oil-repellent agent according to any one of Aspects 1 to 9, wherein the water- and oil-repellent agent is for paper.
Aspect 11: A textile product, comprising the modified heterocyclic ring compound in the water- and oil-repellent agent according to any one of Aspects 1 to 10 adhered thereon.
Aspect 12: A water- and oil-repellent paper, comprising the modified heterocyclic ring compound in the water- and oil-repellent agent according to any one of Aspects 1 to 10 adhered thereon.
Aspect 13: The water- and oil-repellent paper according to Aspect 12, wherein the water- and oil-repellent paper is a food packaging material or a food container.
Aspect 14: A glass, comprising the modified heterocyclic ring compound in the water- and oil-repellent agent according to any one of Aspects 1 to 10 adhered thereon.
Aspect 15: A method of treating the water- and oil-repellent paper according to Aspect 12 or 13, comprising subjecting the paper to an external addition treatment or an internal addition treatment.
Aspect 16: A water dispersion composition, comprising a modified heterocyclic ring compound having a hydrocarbon group having 1 to 40 carbon atoms and a heterocyclic ring; and a surfactant.
Aspect 17: A compound, represented by the formula:

   A(-B-R)ₙ

   wherein
   A is a pyridine ring, a diazine ring, or a triazine ring;
   B is -NR²-, -O-, -C(=O)NR²-, -NR²C(=O)-, -C(=O)O-, -OC(=O)-, -NR²C(=O)NR²-, or -OC(=O)NR²-, wherein R² are identical or different and each a hydrogen atom or a hydrocarbon group having 1 to 10 carbon atoms;
   R is a hydrocarbon group having 1 to 40 carbon atoms and optionally having a substituent; and
   n is a number of 1 to 5.

### Advantageous Effects of Invention

The water- and oil-repellent agent of the present disclosure exhibits at least one of excellent oil-resistance, water-repellency and oil-repellency.

### Description of Embodiments

The water- and oil-repellent agent imparts at least one of the water- and oil-repellency (water-repellency and oil-repellency) and the oil-resistance to a treatment target (substrate) by treating the treatment target with the water- and oil-repellent agent. The oil-resistance includes a function of preventing oil stain into the treatment target, or functions of the oil-repellency, the antifouling property or the like, but functions are not limited thereto.

Here, "oil" refers to fats and oils and organic solvents; the fats and oils include edible fats and oils (plant fatty oils, animal fatty oils, plant fats, animal fats) and industrial fats and oils; the edible fats and oils include salad oils, corn oils, sesame oils, rapeseed oils and olive oils, and industrial fats and oils include castor oils; organic solvents may be polar solvents or nonpolar solvents, and the nonpolar solvents include hexane and hexadecane, but are not limited thereto.

Further, the treatment target may have, in addition to the oil-repellency and/or the oil-resistance, the water-resistance and the water-repellency. The water- and oil-repellent agent may comprise, in addition to the modified heterocyclic ring compound, a liquid medium (water, an organic solvent (for example, chloroform, toluene or acetone) or a mixed solution thereof). The water- and oil-repellent agent may further comprise at least one selected from the group consisting of surfactants, dispersants, blocked isocyanate compounds and additives.

In the present disclosure, a modified heterocyclic ring compound is produced by modifying a heterocyclic ring compound (hereinafter, also referred to as "raw material heterocyclic ring compound") with a modifying agent. The "heterocyclic ring compound" means a compound having a ring having, as a ring constituting atom, a nitrogen atom, an oxygen atom or a sulfur atom. The heterocyclic ring of the raw material heterocyclic ring compound has both of hydrophobicity and hydrophilicity.

The raw material heterocyclic ring compound may be a compound as it is a natural product, or a compound originated from a natural product, but is usually a synthetic product.

The raw material heterocyclic ring compound may be a heterocyclic ring compound having, as a constituting element of the heterocyclic ring, a nitrogen atom, an oxygen atom or a sulfur atom. It is preferable that the heterocyclic ring contain a nitrogen atom as a constituting element. The heterocyclic ring may be a 5-, 6- or 7-membered monocyclic ring, or an 8- to 20-membered condensed ring being a condensate of two or more rings. The heterocyclic ring may be saturated or unsaturated, but is preferably unsaturated. It is especially preferable that the heterocyclic ring be aromatic (a heteroaryl group).

The raw material heterocyclic ring compound is of a low molecular weight, and the molecular weight of the raw material heterocyclic ring compound usually may be 40 to 300, 50 to 250, or 60 to 200. The molecular weight of the modified heterocyclic ring compound may be 80 to 800, 120 to 500, or 150 to 400.

The number of groups introduced by the modifying agent, in the heterocyclic ring of the raw material heterocyclic ring compound (that is, the heterocyclic ring of the modified heterocyclic ring compound), may be 1 to 5, or 2 to 4. 2 or 3 is preferable.

Specific examples of the raw material heterocyclic ring compound (or the backbone of the raw material heterocyclic ring compound) are pyrrolidine, pyrrole, pyridine, pyrazine, pyrimidine, pyridazine, diazine, oxazine, thiazine, triazine, tetrazine, quinoline, quinoxaline, quinazoline, phthalazine, cinnoline, isoquinoline, pteridine, acridine, phenazine, phenanthroline, tetrazole, pyrazole, imidazole, thiazole, oxazole, indazole, benzimidazole, benzotriazole, benzoxazole, benzothiazole, carbazole, purine, triazolopyridazine, triazolopyrimidine, tetrazaindene, oxadiazole, imidazopyridine, pyrrolopyridine, thiadiazolopyridine, azepine, diazepine, thiazepine, dibenzazepine and tribenzazepine. The raw material heterocyclic ring compound may be substituted on carbon atoms of the ring with various substituents (for example, alkyl groups (having 1 to 10 carbon atoms), and halogen atoms such as chlorine, bromine and iodine), and ring-constituting carbon atoms may be bonded to substituents.

The raw material heterocyclic ring compound is preferably a nitrogen-containing heterocyclic ring compound. The "nitrogen-containing heterocyclic ring compound" means a compound having a ring having a nitrogen atom as a constituting atom of the ring. In the "nitrogen-containing heterocyclic ring compound", in addition to the nitrogen atom, an oxygen atom and/or a sulfur atom may constitute the heterocyclic ring.

Preferable examples of the raw material heterocyclic ring compound (or the backbone of the raw material heterocyclic ring compound) may be pyrrole, pyridine, pyrazine, pyrimidine, pyridazine, diazine, oxazine, thiazine, triazine, tetrazine, quinoline, quinoxaline, quinazoline, phthalazine, cinnoline, isoquinoline, pteridine, acridine, phenazine, phenanthroline, tetrazole, pyrazole, imidazole, thiazole, oxazole, indazole, benzimidazole, benzotriazole, benzoxazole, benzothiazole, carbazole, purine, triazolopyridazine, triazolopyrimidine, tetrazaindene, oxadiazole, imidazopyridine, pyrrolopyridine, thiadiazolopyridine, azepine, diazepine, thiazepine, dibenzazepine and tribenzazepine.

The backbone (ring) of the raw material heterocyclic ring compound is preferably a heteroaryl group.

Especially preferable are pyrimidine, pyridazine, pyridine, diazine, triazine, oxazole, oxadiazole, isoxazole, imidazole and pyrazine.

The raw material heterocyclic ring compound react with the modifying agent and the modified heterocyclic ring compound is formed. Examples of synthesis methods of reacting the modifying agent with the raw material heterocyclic ring compound include a method of forming an amino bond, a method of forming an ether bond, a method of forming an ester bond, a method of forming an amido bond, a method of forming a urea bond or a thiourea bond, a method of forming carbamate bond or a thiocarbamate bond, and a method of forming a thiol bond.

A reaction group in the raw material heterocyclic ring compound is a carboxyl group, a hydroxyl group, a chlorine atom, a fluorine atom, a bromine atom, an iodine atom, an amino group, a formyl group, ketone, acid chloride, an azido group, an alkynyl group, an alkenyl group, a thiol group, a cyano group, an amido group or the like.

The substitution ratio of the reaction group (for example, a carboxyl group, a hydroxyl group or a chlorine atom) by the modifying agent is higher than 0, for example, 1% or higher, 3% or higher, 5% or higher, or 10% or higher and more preferably, for example, 15% or higher, 20% or higher, 30% or higher, 80% or higher, 90% or higher, or 95% or higher. The "substitution ratio" means a proportion (%) in which the reaction group present in the structure of the modified heterocyclic ring compound is substituted by the modifying agent.

The ratio of the unmodified reaction group (that is, the residual ratio of the carboxyl group) is lower than 100%, and may be 95% or lower, 90% or lower, 60% or lower, 40% or lower, or 20% or lower. The "residual ratio" means a proportion (%) in which the reaction group present in the structure of the modified heterocyclic ring compound is not substituted by the modifying agent.

Examples of the modifying agent are as follows.
Amine compounds (R¹-NR²H)
Alcohol compounds (R¹-OH)
Isocyanate compounds (R¹-N=C=O)
Isothiocyanate compounds (R¹-N=C=S)
Halogenated compounds (R¹-X)
Epoxy compounds (R³-CHOCH₂)
Aldehyde compounds (R¹CHO)
Ketone compounds (R¹C=OR³)
Acid chloride compounds (R¹COCl)
Acid anhydride compounds (R¹C=OOO=CR¹)
Thiol compounds (R¹-SH)
wherein R¹ is a hydrocarbon group having 1 to 40 carbon atoms and optionally having a substituent;
R² is hydrogen or a hydrocarbon group having 1 to 40 carbon atoms and optionally having a substituent;
R³ is a hydrocarbon group having 1 to 40 carbon atoms and optionally having a substituent; and
X is Cl, Br or I.

Two R¹ groups (or R¹ and R², or R¹ and R³) may together form a ring.

The number of carbon atoms of R¹ may be 3 or larger, 4 or larger, 8 or larger, 10 or larger, 12 or larger, 14 or larger, 16 or larger, 18 or larger, or 20 or larger, and is preferably 8 or larger, or 12 or larger. The number of carbon atoms of the hydrocarbon group may be 40 or smaller, 35 or smaller, 30 or smaller, 25 or smaller, 22 or smaller, 20 or smaller, or 18 or smaller, and is preferably 30 or smaller. The number of carbon atoms may be, for example, 4 to 35, 6 to 30, 8 to 25, or 10 to 22.

The number of carbon atoms of R² may be 1 to 40, 1 to 10, or 1 to 4.

The number of carbon atoms of R³ may be 2 or larger, 4 or larger, 8 or larger, 10 or larger, 12 or larger, 14 or larger, 16 or larger, 18 or larger, or 20 or larger, and is preferably 8 or larger, or 12 or larger. The number of carbon atoms of the hydrocarbon group may be 40 or smaller, 35 or smaller, 30 or smaller, 25 or smaller, 22 or smaller, 20 or smaller, or 18 or smaller, and is preferably 30 or smaller. The number of carbon atoms may be, for example, 4 to 35, 6 to 30, 8 to 25, or 10 to 22.

The R¹, R² and R³ are a saturated or unsaturated hydrocarbon group optionally having a substituent, preferably an aliphatic hydrocarbon group optionally having a substituent and more preferably an aliphatic hydrocarbon group. The hydrocarbon group may be linear, branched or cyclic, and is preferably linear or branched, more preferably linear. R¹, R² and R³ may be, for example, a linear or branched alkyl group.

By reaction of the raw material heterocyclic ring compound with the modifying agent, various bonds (monovalent or divalent bonding groups or spacers), for example, -NH- (or -NR²-) , -O-, -C(=O)NH- (or -C(=O)NR²-), -NHC(=O)- (or - NR²C(=O)-), -C(=O)O-, -OC(=O)-, -NR²C(=O)NR²-, or - OC(=O)NR²-, are formed.

The modified heterocyclic ring compound may be a compound represented by the formula:

A(-B-R)ₙ

Wherein
A is a residue after elimination of a reactive group from the raw material heterocyclic ring compound, or an optionally substituted heterocyclic ring;
B is a bonding group;
R is a hydrocarbon group having 1 to 40 carbon atoms and optionally having a substituent; and
n is a number of 1 to 5.

A is a residue after elimination of a reactive group from the raw material heterocyclic ring compound, or a heterocyclic ring (that is, the backbone of the raw material heterocyclic ring compound). A is preferably a heteroaryl group.

Examples of the reactive group in A are a carboxyl group, a hydroxyl group, a chlorine atom, a fluorine atom, a bromine atom, an iodine atom, an amino group, a formyl group, ketone, acid chloride, an azido group, an alkynyl group, an alkenyl group, a thiol group, a cyano group and an amido group.

Examples of the bonding group are -NH- (or -NR²-), -O-, - C(=O)NH- (or -C(=O)NR²-), -NHC(=O)- (or -NR²C(=O)-), - C (=O) O-, -OC (=O) -, -NR²C(=O)NR²-, or -OC(=O)NR²-. R² are identical or different, and each may be hydrogen or a hydrocarbon group (for example, alkyl group) having 1 to 10 or 1 to 4 carbon atoms and optionally having a substituent.

The number of carbon atoms of R is 1 to 40, and may be 2 or larger, 4 or larger, 8 or larger, 10 or larger, 12 or larger, 14 or larger, 16 or larger, 18 or larger, or 20 or larger, and is preferably 8 or larger, or 12 or larger. The number of carbon atoms of the hydrocarbon group may be 40 or smaller, 35 or smaller, 30 or smaller, 25 or smaller, 22 or smaller, 20 or smaller, or 18 or smaller, and is preferably 30 or smaller. The number of carbon atoms may be, for example, 4 to 35, 6 to 30, 8 to 25, or 10 to 22.

R is a saturated or unsaturated hydrocarbon group optionally having a substituent, preferably an aliphatic hydrocarbon group optionally having a substituent, and more preferably an aliphatic hydrocarbon group. The hydrocarbon group may be linear, branched or cyclic, and is preferably linear or branched, and more preferably linear. R may be, for example, a linear or branched alkyl group.

n may be 1 to 4, or 2 or 3. n is preferably 2 or 3.

A compound represented by the formula:

A(-B-R)ₙ

wherein
A is a pyridine ring, a diazine ring or a triazine ring;
B is -NR²-, -O-, -C(=O)NR²-, -NR²C(=O)-, -C(=O)O-, -OC(=O)-, -NR²C(=O)NR²-, or -OC(=O)NR²-, wherein R² are identical or different and each a hydrogen atom or a hydrocarbon group having 1 to 10 carbon atoms;
R is a hydrocarbon group having 1 to 40 carbon atoms and optionally having a substituent; and
n is a number of 1 to 5,
is a novel compound.
   A is preferably a pyridine ring or a triazine ring.
   B is preferably -C(=O)NR²- or -NR²C(=O)-.
   R² may be a hydrogen atom or a hydrocarbon group (for example, an alkyl group) having 1 to 10 carbon atoms (or having 1 to 4 carbon atoms).
   n may be 1 to 4, or 2 or 3. n is preferably 2 or 3.

### <Method of forming an amino bond>

An amino group can be introduced to the raw material heterocyclic ring compound. As the modifying agent, an amine compound (R¹-NR²H) can be used.

There are methods thereof including:
a method in which the amine compound is made to act directly on a heterocyclic ring compound having a leaving group such as a chlorine atom;
a method in which the amine compound is introduced to a heterocyclic ring compound having a leaving group such as a chlorine atom in the presence of palladium, a ligand or the like by a coupling reaction; and
a method in which an amino bond is formed by reducing a heterocyclic ring-directly bonded amide compound.

### <Method of forming an ether bond>

An ether group is introduced to the raw material heterocyclic ring compound. As the modifying agent, a halogen compound (particularly, alkyl halide compound)(for example, R¹-X), or an epoxy compound (for example, R³-CHOCH₂) can be used.

The ether bond is formed, for example, by reaction of an alkyl halide compound or an epoxy compound with a hydroxyl group of the raw material heterocyclic ring compound.

Synthesis methods of replacing a hydrogen atom of the hydroxyl group of the raw material heterocyclic ring compound by an alkyl group include a method in which a hydroxyl group-containing heterocyclic ring compound is reacted with an alkaline aqueous solution of an alkyl halide compound (for example, sodium hydroxide, potassium hydroxide), and a method in which a basic compound and an alkyl halide dissolved in an organic solvent are reacted therein.

An example of the alkyl halide compound may be a compound represented by

CₙH₂ₙ₊₁-X

wherein n is 1 to 40, or 1 to 30; and X is Cl, Br or I.

Specific examples of the alkyl halide compound include methyl halides, ethyl halides, propyl halides, butyl halides, pentyl halides, hexyl halides, heptyl halides, octyl halides, nonyl halides, decyl halides, undecyl halides, dodecyl halides, tridecyl halides, tetradecyl halides, pentadecyl halides, hexadecyl halides, heptadecyl halides, octadecyl halides, nonadecyl halides, icosyl halides, henicosyl halides, docosyl halides and tricosyl halides.

Examples of the epoxy compound are linear epoxy compounds (for example, CₙH₂ₙ₊₁-CHOCH₂ (n is 1 to 40, or 1 to 30) ) and alicyclic epoxy compounds (for example, CₙH₂ₙ-CHOCH (n is 3 to 15, or 3 to 10)).

Specific examples of the epoxy compound include propylene oxide, 1,2-epoxybutane, 1,2-epoxypentadecane, 1,2-epoxyhexane, 1,2-epoxyheptane, 1,2-epoxyoctane, 1,2-epoxynonane, 1,2-epoxydecane, 1,2-epoxyundecane, 1,2-epoxydodecane, 1,2-epoxytridecane, 1,2-tetradecane, 1,2-epoxypentadecane, 1,2-epoxyhexadecane, 1,2-epoxyheptadecane, 1,2-epoxyoctadecane, 1,2-epoxynonadecane, 1,2-epoxyeicosane, 1,2-epoxyhenicosane, and 1,2-epoxydocosane.

### <Method of forming an ester bond>

By using an alcohol compound (R¹-OH), an ester bond is formed with the carboxyl group of the raw material heterocyclic ring compound.

Examples of synthesis methods of converting a carboxyl group to an ester bond include:
(a) a method of forming an ester bond by using a raw material heterocyclic ring compound, the alcohol compound (R¹-OH) and an acid catalyst;
(b) a method of forming an ester bond by using a raw material heterocyclic ring compound, the alcohol compound (R¹-OH) and a condensing agent; and
(c) a method of forming an ester bond by converting a raw material heterocyclic ring compound to an acyl compound and thereafter using the alcohol compound (R¹-OH),
(in each method, as required, a solvent is added).

In the alcohol compound (R¹-OH), R¹ has the same meaning as in the above.

An example of the alcohol compound may be an aliphatic alcohol.

Examples of the aliphatic alcohol include

(CH₃)_{q}C(H)_{3-q}(CH₂)ₚOH

wherein p is 0 to 30, and q is 0 to 3.

Specific examples of aliphatic alcohols include CH₃OH, CH₃CH₂OH, CH₃(CH₂)₂OH, CH₃(CH₂)₃OH, CH₃(CH₂)₄OH, CH₃(CH₂)₅OH, CH₃(CH₂)₆OH, CH₃(CH₂)₇OH, CH₃(CH₂)₈OH, CH₃(CH₂)₉OH, CH₃(CH₂)₁₀OH, CH₃(CH₂)₁₁OH, CH₃(CH₂)₁₂OH, CH₃(CH₂)₁₃OH, CH₃(CH₂)₁₄OH, CH₃(CH₂)₁₅OH, CH₃(CH₂)₁₆OH, CH₃(CH₂)₁₇OH, CH₃(CH₂)₁₈OH, CH₃(CH₂)₁₉OH, CH₃(CH₂)₂₀OH, CH₃(CH₂)₂₁OH, CH₃(CH₂)₂₂OH, CH₃(CH₂)₂₃OH, (CH₃)₂CHOH, (CH₃)₃COH, (CH₃)₂CHCH₂OH, (CH₃)₃CCH₂OH, (CH₃)₂CH(CH₂)₂OH, (CH₃)₃C(CH₂)₁₄OH, (CH₃)₂CH(CH₂)₁₅OH, (CH₃)₃C(CH₂)₁₈OH and (CH₃)₂CH(CH₂)₁₈OH.

Examples of the acid catalyst may be Bronsted acids such as sulfuric acid, tosylic acid, hydrochloric acid and nitric acid, and Lewis acids such as tetraisopropoxytitanium and boron trifluoride.

Examples of the condensing agent may be phosphonium-based condensing agents (PyBOP, BOP and the like), carbodiimidazole-based condensing agents (DCC, EDCl and the like), uronium-based condensing agents (COMU, HATU and the like), and imidazole-based condensing agents (CDI and the like).

### <Method of forming an amido bond>

An amido bond is formed by reacting a carboxyl group of the raw material heterocyclic ring compound. The amido bond can be formed by reacting the raw material heterocyclic ring compound with an amine compound (R¹-NR²H) by using a condensing agent, or converting a carboxyl group of the raw material heterocyclic ring compound to an active species such as an acid chloride or an acid azide, and thereafter making the active species to interact with the amine compound.

Examples of methods of converting a carboxyl group of the raw material heterocyclic ring compound to an amido group include;
a method in which the carboxyl group of the raw material heterocyclic ring compound is converted to an active species such as an acyl compound (particularly, acid chloride) or an acid azide, and thereafter, mixing the active species with an amine compound (R¹-NR²H) to thereby form an amido bond; and
a method of forming an amido bond by using a heterocyclic ring compound, an amine compound (R¹-NR²H) and a condensing agent.

Specific examples of the amine compound include CH₃NH₂, CH₃CH₂NH₂, CH₃(CH₂)₂NH₂, CH₃(CH₂)₃NH₂, CH₃(CH₂)₄NH₂, CH₃(CH₂)₅NH₂, CH₃(CH₂)₆NH₂, CH₃(CH₂)₇NH₂, CH₃(CH₂)₈NH₂, CH₃(CH₂)₉NH₂, CH₃(CH₂)₁₀NH₂, CH₃(CH₂)₁₁NH₂, CH₃(CH₂)₁₂NH₂, CH₃(CH₂)₁₃NH₂, CH₃(CH₂)₁₄NH₂, CH₃(CH₂)₁₅NH₂, CH₃(CH₂)₁₆NH₂, CH₃(CH₂)₁₇NH₂, CH₃(CH₂)₁₈NH₂, CH₃(CH₂)₁₉NH₂, CH₃(CH₂)₂₀NH₂, CH₃(CH₂)₂₁NH₂, CH₃(CH₂)₂₂NH₂, CH₃(CH₂)₂₃NH₂, (CH₃)₂CHNH₂, (CH₃)₃CNH₂, (CH₃)₂CHCH₂NH₂, (CH₃)₃CCH₂NH₂, (CH₃)₂CH(CH₂)₂NH₂, (CH₃)₃C(CH₂)₁₄NH₂, (CH₃)₂CH(CH₂)₁₅NH₂, (CH₃)₃C(CH₂)₁₈NH₂ and (CH₃)₂CH(CH₂)₁₈NH₂ .

Specific examples of the isocyanate compound include CH₃NCO, CH₃CH₂NCO, CH₃(CH₂)₂NCO, CH₃(CH₂)₃NCO, CH₃(CH₂)₄NCO, CH₃(CH₂)₅NCO, CH₃(CH₂)₆NCO, CH₃(CH₂)₇NCO, CH₃(CH₂)₈NCO, CH₃(CH₂)₉NCO, CH₃(CH₂)₁₀NCO, CH₃(CH₂)ₙNCO, CH₃(CH₂)₁₂NCO, CH₃(CH₂)₁₃NCO, CH₃(CH₂)₁₄NCO, CH₃(CH₂)₁₅NCO, CH₃(CH₂)₁₆NCO, CH₃(CH₂)₁₇NCO, CH₃(CH₂)₁₈NCO, CH₃(CH₂)₁₉NCO, CH₃(CH₂)₂0NCO, CH₃(CH₂)₂₁NCO, CH₃(CH₂)₂₂NCO, CH₃(CH₂)₂₃NCO, (CH₃)₂CHNCO, (CH₃)₃CNCO, (CH₃)₂CHCH₂NCO, (CH₃)₃CCH₂NCO, (CH₃)₂CH(CH₂)₂NCO, (CH₃)₃C(CH₂)₁₄NCO, (CH₃)₂CH(CH₂)₁₅NCO, (CH₃)₃C(CH₂)₁₈NCO and (CH₃)₂CH(CH₂)₁₈NCO.

Specific examples of the isothioisocyanate compound include CH₃NCS, CH₃CH₂NCS, CH₃(CH₂)₂NCS, CH₃(CH₂)₃NCS, CH₃(CH₂)₄NCS, CH₃(CH₂)₅NCS, CH₃(CH₂)₆NCS, CH₃(CH₂)₇NCS, CH₃(CH₂)₈NCS, CH₃(CH₂)₉NCS, CH₃(CH₂)₁₀NCS, CH₃(CH₂)₁₁NCS, CH₃(CH₂)₁₂NCO, CH₃(CH₂)₁₃NCO, CH₃(CH₂)₁₄NCO, CH₃(CH₂)₁₅NCO, CH₃(CH₂)₁₆NCS, CH₃(CH₂)₁₇NCS, CH₃(CH₂)₁₈NCS, CH₃(CH₂)₁₉NCS, CH₃(CH₂)₂₀NCS, CH₃(CH₂)₂₁NCS, CH₃(CH₂)₂₂NCS, CH₃(CH₂)₂₃NCS, (CH₃)₂CHNCS, (CH₃)₃CNCS, (CH₃)₂CHCH₂NCS, (CH₃)₃CCH₂NCS, (CH₃)₂CH(CH₂)₂NCS, (CH₃)₃C(CH₂)₁₄NCS, (CH₃)₂CH(CH₂)₁₅NCS, (CH₃)₃C(CH₂)₁₈NCS and (CH₃)₂CH(CH₂)₁₈NCS.

### <Method of forming a urea bond or a thiourea bond>

A urea bond or a thiourea bond can be formed by mixing an amino group of the raw material heterocyclic ring compound with an isocyanate compound (R¹-N=C=O) or an isothiocyanate compound (R¹-N=C=S).

Specific examples of the isocyanate compound (R¹-N=C=O) and the isothiocyanate compound (R¹-N=C=S) are the same as in the above.

### <Method of forming a carbamate bond or a thiocarbamate bond>

A carbamate bond or a thiocarbamate bond can be formed by mixing a hydroxyl group directly bonded to the raw material heterocyclic ring compound or a hydroxyl group through a linker with an isocyanate compound (R¹-N=C=O) or an isothiocyanate compound (R¹-N=C=S).

Specific examples of the isocyanate compound (R¹-N=C=O) and the isothiocyanate compound (R¹-N=C=S) are the same as in the above.

In the present disclosure, the modified heterocyclic ring compound is preferably a modified heterocyclic ring compound modified with a primary amine.

### <Water- and oil-repellent agent>

The modified heterocyclic ring compound, since having water-repellency and oil-repellence and/or oil-resistance, can be used as the "water- and oil-repellent agent". The water- and oil-repellent agent may be a composition containing the modified heterocyclic ring compound, or the water- and oil-repellent agent may be composed only of the modified heterocyclic ring compound. The water- and oil-repellent agent may further have water-resistance. The water- and oil-repellent agent may contain, in addition to the modified heterocyclic ring compound, a liquid medium (water, an organic solvent or a mixed solution thereof). The water- and oil-repellent agent may further contain at least one selected from the group consisting of surfactants, dispersants, cross-linking agents such as blocked isocyanate compounds, and additives.

The amount of the modified heterocyclic ring compound may be, with respect to the water- and oil-repellent agent, 0.01% by weight or larger, 0.1% by weight or larger, 1% by weight or larger, 10% by weight or larger, 20% by weight or larger or 30% by weight or larger. The amount of the modified heterocyclic ring compound may be, with respect to the water- and oil-repellent agent, 100% by weight or smaller, 95% by weight or smaller, 90% by weight or smaller or 80% by weight or smaller.

The water- and oil-repellent agent may contain a liquid medium, particularly, an aqueous medium (water, or a mixture of water and an organic solvent). The water- and oil-repellent agent is usually a solution or a dispersion. The solution is a solution in which a polymer is dissolved in an organic solvent or an aqueous medium. The dispersion is an aqueous dispersion in which a polymer is dispersed in an aqueous medium.

Examples of the organic solvent are esters (for example, esters having 2 to 40 carbon atoms, specifically, ethyl acetate, butyl acetate), ketones (for example, ketones having 2 to 40 carbon atoms, specifically, acetone, methyl ethyl ketone, diisobutyl ketone), alcohols (for example, alcohols having 1 to 40 carbon atoms, specifically, isopropyl alcohol), aromatic solvents (for example, toluene and xylene), petroleum solvents (for example, alkanes having 5 to 10 carbon atoms, specifically, naphtha, kerosine), and alkyl halides (for example, alkyl halides having 1 to 40 carbon atoms, specifically, chloroform).

The liquid medium may be water singly, an organic solvent singly, or a mixture of water and a (water-miscible) organic solvent. The amount of the organic solvent may be, with respect to the liquid medium, 30% by weight or smaller, for example, 10% by weight or smaller (preferably 0.1% by weight or larger). The liquid medium is preferably water singly.

The amount of the liquid medium may be, with respect to the water- and oil-repellent agent, 60% by weight or larger, 80% by weight or larger or 90% by weight or larger, and the amount of the liquid medium may be smaller than 100% by weight, 99% by weight or smaller, 95% by weight or smaller, 90% by weight or smaller or 80% by weight or smaller.

### <Surfactant or dispersant>

The water- and oil-repellent agent does not necessarily contain a surfactant (emulsifier) or a dispersant, or may contain. During the reaction time or after the reaction, a small amount (for example, with respect to 100 parts by mass of the modified heterocyclic ring compound, 0.01 to 100 parts by mass or 0.01 to 50 parts by mass, for example, 0.1 to 30 parts by mass) of the surfactant or the dispersant may be added. When a surfactant or a dispersant is added, the stability of a water dispersion is usually improved.

It is preferable that the surfactant contain one or more kinds of surfactants selected from cationic surfactants, nonionic surfactants, anionic surfactants and amphoteric surfactants; and it is more preferable to use a nonionic surfactant, a cationic surfactant or a combination of a nonionic surfactant and a cationic surfactant.

The surfactant may be a combination of each one kind or each two or more kinds of nonionic surfactants, cationic surfactants, anionic surfactants and amphoteric surfactants.

The surfactant or the dispersant may be added in an amount of, with respect to 100 parts by mass of the modified heterocyclic ring compound, 0.01% by weight or larger, 0.1% by weight or larger, 1% by weight or larger, 5% by weight or larger, 10% by weight or larger, 30% by weight or larger or 50% by weight or larger, and then, 100% by weight or smaller, 95% by weight or smaller, 90% by weight or smaller or 70% by weight or smaller. The addition of the surfactant or the dispersant usually improves the stability of a water dispersion.

Examples of the nonionic surfactant (emulsifier) or dispersant include glycerol fatty acid esters, sorbitan fatty acid esters, sucrose fatty acid esters, polyoxyethylene alkyl ethers, polyoxyethylene alkyl phenyl ethers, polyoxyethylene polyoxypropylene glycols, fatty acid polyethylene glycols, fatty acid polyoxyethylene sorbitan and fatty acid alkanolamides.

Examples of the cationic surfactant (emulsifier) or dispersant include alkylamine salts and quaternary ammonium salts; the alkylamine salts include monoalkylamine salts, dialkylamine salts and trialkylamine salts, and the quaternary ammonium salts include a trimethylammonium chloride salt, dialkyldimethylammonium chloride salts and alkyl benzalkonium chloride salts.

Examples of the anionic surfactant (emulsifier) or dispersant include carboxylate salts, sulfonate salts, sulfate ester salts and phosphate ester salts; and examples of the carboxylate salts include aliphatic monocarboxylate salts and alkyl ether carboxylate salts; examples of the sulfonate salts include dialkylsulfosuccinate salts, alkanesulfonate salts, alkylbenzenesulfonate salts and alkylnaphthalenesulfonate salts; examples of the sulfate ester salts include alkylsulfate salts and fat-and-oil sulfate ester salts; and examples of the phosphate ester salts include alkylphosphate salts and polyoxyethylene alkyl ether phosphate salts.

Examples of the amphoteric surfactant (emulsifier) or dispersant include alkyl betaines, fatty acid amide propyl betaines, 2-alkyl-N-carboxymethyl-N-hydroxyethylimidazolium salts, alkyl diethylene triaminoacetatic acids, dialkyl diethylene triaminoacetatic acids and alkylamine oxides.

When the surfactant (emulsifier) or the dispersant is added during or after the reaction of the modified heterocyclic ring compound, heating may be carried out. After the dispersant is added to the modified heterocyclic ring compound, they may be heated and melted and then dispersed; or they may be dispersed or dissolved by adding a solution (preferably, water) under further heating. The heating temperature may be 40°C or higher, 60°C or higher or 80°C or higher.

The water- and oil-repellent agent does not necessarily contain a blocked isocyanate compound, or may contain. The blocked isocyanate compound may be added before the substitution reaction of the carboxyl group, or may be added after the reaction (for example, before a curing process).

Preferable blocked isocyanate compounds are blocked isocyanates such as oxime blocked toluene diisocyanate, blocked hexamethylene diisocyanate and blocked diphenylmethane diisocyanate.

The amount of the blocked isocyanate compound may be, with respect to 100 parts by weight of the modified heterocyclic ring compound, 15 parts by weight or smaller, 10 parts by weight or smaller, 7.5 parts by weight or smaller, 5 parts by weight or smaller or 2.5 parts by weight or smaller.

The water- and oil-repellent agent may contain additives. Examples of the additives are binder resins, dispersants, water-resistant agents, oil-resistant agents, water-repellent agent, oil-repellent agent, drying rate adjusters, cross-linking agents, film forming assistants, compatibilizers, antifreezing agents, viscosity adjusters, ultraviolet absorbents, antioxidants, pH adjusters, antifoaming agents, texture modifiers, slippage modifiers, antistatic agents, hydrophilizing agents, antibacterial agents, antiseptic agents, insect repellents, fragrant agents, flame retarders, retention aids, sizing agents, paper strength additives and fillers.

Examples of the retention aids include aluminum sulfate, acrylic polymers, starch, modified starch, cellulose, modified cellulose and silica.

Examples of the sizing agents include rosin-based sizing agents, alkyl ketene dimers (AKD), alkenyl succinic anhydrides (ASA), polyvinyl alcohols (PVA), modified starch, styrene·acrylic copolymers and styrene -methacrylic copolymers.

Examples of the pH adjusters include lactic acid, carbon dioxide, succinic acid, gluconic acid, citric acid, trisodium citrate, phosphoric acid, potassium carbonate and sodium hydrogencarbonate.

Examples of the fillers include talc, kaolin, calcium carbonate, titanium oxide and barium sulfate.

Examples of the paper strength additives include urea formaldehyde resins, melamine formaldehyde resins, polyamide polyamine epichlorohydrins (PAE), polyvinylamines (PVAm), modified starch, polyacrylamides and polyvinyl alcohols.

The amount of the additives is, with respect to 100 parts by weight of the modified heterocyclic ring compound, larger than 0 and preferably 0.1 part by weight or larger and more preferably 1 part by weight or larger, and 100 parts by weight or smaller, more preferably 80 parts by weight or smaller and still more preferably 50 parts by weight or smaller.

### <Properties of the water- and oil-repellent agent and the modified heterocyclic ring compound>

The melting point of side chains in the modified heterocyclic ring compound, preferably, is 0°C or higher or exhibits no melting point, and is preferably 20°C or higher, 40°C or higher or 60°C or higher. Then, the melting point of the side chains is preferably 180°C or lower, 140°C or lower or 120°C or lower. With the melting point of the side chains in the above range, the resistance to temperature is improved and in particular, from the viewpoint of high-temperature oil-resistance and the like, the melting point being in the above range is preferable.

The contact angle of n-hexadecane against the water- and oil-repellent agent (on a glass substrate) may be 5° or larger, 10° or larger or 15° or larger, preferably 20° or larger or 25° or larger, more preferably 30° or larger and most preferably 35° or larger or 40° or larger. With the contact angle of n-hexadecane in the above range, the water- and oil-repellent agent is excellent in the liquid-repellency, and in particular, from the viewpoint of the oil-resistance and the like, the contact angle being in the above range is preferable.

The melting point of the modified heterocyclic ring compound is preferably 20°C or higher, more preferably 40°C or higher, still more preferably 60°C or higher and most preferably 80°C or higher. Then, the melting point of the modified heterocyclic ring compound is preferably 200°C or lower, 180°C or lower or 160°C or lower. With the melting point of the modified heterocyclic ring compound in the above range, the coatability when the oil-resistant agent is applied to a texture product, and the resistance to temperature are improved and in particular, from the viewpoint of the high-temperature oil-resistance, and the like, the melting point being in the above range is preferable.

The glass transition temperature of the modified heterocyclic ring compound, preferably, is 0°C or higher or exhibits no glass transition temperature, more preferably, is 20°C or higher or exhibits no glass transition temperature, still more preferably, is 40°C or higher or exhibits no glass transition temperature, and most preferably, is 60°C or higher or exhibits no glass transition temperature. Then, the glass transition temperature of the modified heterocyclic ring compound is preferably 180°C or lower, 140°C or lower or 120°° or lower. With the glass transition temperature of the modified heterocyclic ring compound in the above range, the coatability when the oil-resistant agent is applied to a texture product, and the resistance to temperature are improved and in particular, from the viewpoint of the oil-resistance, and the like, the glass transition temperature being in the above range is preferable.

The viscosity of the water- and oil-repellent agent (solution or dispersion) of 14.8 mg/mL in concentration is preferably 3 cP or higher, 5 cP or higher, 7 cP or higher, or 10 cP or higher. Then, the viscosity of the water- and oil-repellent agent (solution or dispersion)of 14.8 mg/mL in concentration is preferably 1,000 cP or lower, 500 cP or lower, or 100 cP or lower. With the viscosity of the solution (dispersion) in the above range, the coatability when the oil-resistant agent is applied to a texture product is improved and in particular, from the viewpoint of the oil-resistance, and the like, the viscosity being in the above range is preferable.

The air permeability of a treated paper, which is fabricated by three times carrying out an operation in which the water- and oil-repellent agent (a solution or a dispersion) in a concentration of 14.8 mg/mL is coated by a Baker applicator set at 0 mil on a base paper of a paper of 0.58 g/cm³ in paper density and 45 g/m² in basis weight, and dried, and annealing the resultant at a temperature of 70°C to 100°C for 10 min, is preferably 10,000 s/100 cc or lower, more preferably 5,000 s/100 cc or lower, still more preferably 3,000 s/100 cc or lower and most preferably 1,000 s/100 cc or lower.

### <Specific examples of the water- and oil-repellent agent>

The water- and oil-repellent agent can be used as various types of agents, such as a water-repellent, an oil-repellent, a water-resistant agent, an oil-resistant agent, a soil-resistant agent, a soil release agent, a release agent and a mold release agent, and also as a component of these.

The water- and oil-repellent agent can be used as a treatment agent such as an external treatment agent (surface-treating agent) or an internal treatment agent, or as a component thereof.

By treating a substrate with the water- and oil-repellent agent, the modified heterocyclic ring compound can form a surface coating structure on the substrate surface.

The surface coating structure can be formed by applying the water- and oil-repellent agent to a treatment target (substrate) by a conventionally well-known method to adhere the oil-resistant agent on the substrate surface. A method is usually adopted in which the modified heterocyclic ring compound is dispersed in and diluted with an organic solvent or water, and the resultant is made to adhere on the surface of the treatment target by a well-known method such as dip coating, spray coating or foam coating, and dried. Then, as required, the modified heterocyclic ring compound may be applied together with a suitable cross-linking agent (for example, a blocked isocyanate compound) and then cured. Further, it is also possible to add and concurrently use an insect repellent, a softening agent, an antibacterial agent, a flame retarder, an antistatic agent, a coating material fixing agent, a wrinkle-resistant agent, a sizing agent, a paper strength additive and the like.

Treatment targets to be treated with the water- and oil-repellent agent include textile products, stone, filters (for example, electrostatic filters), dustfree masks, parts (for example, gas diffusion electrodes and gas diffusion supports) of fuel cells, glass, wood, leather, fur, asbestos, bricks, cement, metals and oxides, ceramic products, plastics, painted surfaces and plaster.

As the textile products, various types thereof can be cited, but examples thereof include fabric products and paper products.

Examples of the fabric products include animal and plant natural fibers such as cotton, hemp, wool, and silk, synthetic fibers such as polyamide, polyester, polyvinyl alcohol, polyacrylonitrile, polyvinyl chloride and polypropylene, semisynthetic fibers such as rayon and acetate fibers, inorganic fibers such as glass fibers, carbon fibers and asbestos fibers, and mixed fibers thereof. The fabric products include woven fabrics, knitted fabrics and nonwoven fabrics, and fabrics in clothing forms and carpets; and some treatment may be carried out on fibers, threads and intermediate textile products (for example, slivers or slubbings) in the state before being made into the fabrics.

Examples of the paper products include papers composed of bleached or unbleached chemical pulps, such as kraft pulp and sulfite pulp, bleached or unbleached high yield pulps, such as groundwood pulp, mechanical pulp and thermomechanical pulp, and papers composed of waste paper pulps and the like such as news, magazine waste papers, corrugated fiberboard waste pulps or deinked waste papers, containers composed of papers, and formed articles composed of papers. Specific examples of the paper products are wrapping papers for food, gypsum liner boards, base coated papers, mechanical papers, general use liners and corrugating media, neutral pure white machine glazed papers, neutral liners, rust-preventive liners and metal-laminated papers, kraft papers, neutral printing writing papers, neutral base coated papers, neutral PPC papers, neutral thermosensitive papers, neutral pressure sensitive papers, neutral inkjet papers and neutral specialty papers for communication, and mold papers (mold containers). The modified heterocyclic ring compound of the present disclosure is excellent in the oil-resistance (for example, high-temperature oil-resistance), and hence is suitably used in applications requiring the oil-resistance, particularly for food packaging materials and food containers.

The water- and oil-repellent agent can be applied to fibrous substrates (for example, textile products, and textile raw materials such as pulps) by any of methods known for treating textile products with a liquid. The fibrous substrate means both of a textile product and a textile raw material. When the textile product is a fabric, the fabric may be dipped in a solution (or a dispersion), or a solution (or a dispersion) may be adhered or sprayed on the fabric. The treatment may be an external addition treatment or an internal addition treatment. When the textile product is a paper, a solution (or a dispersion) may be coated on the paper, or a solution (or a dispersion) may be adhered or sprayed on the paper, or alternatively, a solution (or a dispersion) may be mixed with a pulp slurry before papermaking for the treatment. The treatment may be an external addition treatment or an internal addition treatment.

The water- and oil-repellent agent may be applied to textile products (particularly, papers, fabrics and the like) previously formed, or may also be applied at various stages of papermaking, for example, during the paper drying period.

For example, the fibrous substrate may be a leather. In order to render the leather hydrophobic and oleophobic, the water- and oil-repellent agent may be applied as an aqueous solution or an aqueous emulsion to the leather at various stages in the leather processing period, for example, during a wet processing of the leather, or during the leather finishing period.

In the case where a treatment target is a glass or a metal, it is preferable that the water- and oil-repellent agent be applied on the surface of the treatment target and an active component (modified heterocyclic ring compound) of the water- and oil-repellent agent be adhered on the surface of the treatment target.

The water- and oil-repellent agent can be used also be as an external mold release agent. For example, the surface of a substrate can easily be peeled off from another surface (the other surface of the substrate, or the surface of another substrate).

### <Treatment>

The "treatment" means applying the water- and oil-repellent agent to a treatment target by dipping, spraying, coating or the like. By the treatment, the modified heterocyclic ring compound being an active component of the water- and oil-repellent agent penetrates into the inside of the treatment target and/or adheres on the surface of the treatment target.

In order to develop the oil-resistance, the treatment target (substrate) treated is dried, preferably heated, for example, at a temperature of not lower than the glass transition temperature (Tg) of the modified heterocyclic ring compound, for example, 40°C or higher, 60°C or higher or 80°C or higher, or at a temperature of 250°C or lower or 200°C or lower. By the treatment at a temperature of not lower than Tg of the modified heterocyclic ring compound, the arrangement of side chains is induced in some cases. The treatment temperature is more preferably a temperature of the melting point (Tm) or higher. By the treatment at a temperature of not lower than Tm of the modified heterocyclic ring compound, the substrate surface is coated with the modified heterocyclic ring compound. Thereby, a surface coating structure excellent in hydrophobicity can be formed.

In the case of the treatment with the water- and oil-repellent agent in a liquid state, in order to enhance the desolventizing (dehydrating) property or the adhesion between the modified heterocyclic ring compound and the substrate, the fibrous substrate treated may be pressurized, and the treatment is carried out at a pressure of 0.1 MPa or higher, preferably 0.15 MPa or higher and more preferably 0.2 MPa or higher.

### <Additive for paper>

The water- and oil-repellent agent can suitably be used particularly for an additive for paper. The additive for paper containing the water- and oil-repellent agent can be used, in addition to a water- and oil-repellent agent, as a water-resistant agent, a water-repellent, and/or an oil-repellent. It is preferable that the additive for paper be in a form of solution, emulsion or aerosol. The additive for paper may contain the modified heterocyclic ring compound and a medium (for example, a liquid medium such as an organic medium or water), and is preferably a water dispersion of the modified heterocyclic ring compound. In the additive for paper, the concentration of the modified heterocyclic ring compound may be, for example, 0.01 to 50% by weight. The additive for paper may not contain a surfactant.

The removal of the solution (organic solvent, water) contained in the additive for paper can be carried out by heating the modified heterocyclic ring compound solution (dispersion) at a temperature of 40°C or higher, 60°C or higher or 80°C or higher, and a temperature of 250°C or lower or 200°C or lower.

The additive for paper can be used to treat (for example, surface treatment) a paper base. The additive for paper can be applied to a treatment target by a conventionally well-known method. Usually, a method (surface treatment) is adopted in which the additive for paper is dispersed in and diluted with an organic solvent or water, and adhered on the surface of a treatment target by a well-known method such as dip coating, spray coating or foam coating, and dried. Paper bases of the treatment target include papers, containers composed of a paper, formed articles composed of a paper (for example, pulp mold). The modified heterocyclic ring compound of the present disclosure adheres well on paper bases. Here, "adhere" refers to the formation of a physical bond or a chemical bond. By making the modified heterocyclic ring compound to adhere on paper bases, oilproof papers are obtained.

Hitherto, embodiments have been described, but it is to be understood that modes and details may be modified and changed variously without departing from the gist and the scope of the claims.

### Examples

Then, the present disclosure will be described specifically by way of Examples. However, the present disclosure is not any more limited to these descriptions. Hereinafter, parts, % and ratio denote, unless otherwise specified, parts by weight, % by weight and weight ratio.

### Test methods used in the below were as follows.

### Fabrication of a treated paper

A 14.9-mg/cm³ chloroform solution of a compound was coated three times with a Baker applicator set at a gap of 0 mil on a thin paper of 52 g/m² in water-resistance (Cobb value), 45 g/m² in basis weight, and 0.60 g/m³ in density, and thereafter annealed at 70°C for 10 min to thereby fabricate a treated paper. For the preparation of the solution of the coating liquid, chloroform was used. In the case where the compound was insoluble to chloroform, an organic solvent such as toluene and acetone was used.

### KIT test (oil-resistance)

The oil-resistance was measured by a 3M Kit Test (TAPPI T-559 cm-02). The 3M Kit Test method involved placing a test oil containing castor oil, toluene and heptane blended therein on a surface of the treated paper, and after 15 s, wiping out the test oil and then, evaluating the oil-resistance by checking the presence/absence of oil stain into the treated paper. The test was carried out by using test oils of kit Nos. 1 to 6, and the maximum kit number of the oil exhibited no staining was used as an evaluation result of the oil-resistance.

### Corn oil-resistance test (oil-resistance)

A corn oil is placed on a surface of the treated paper, and after 15 s, wiped out and then, the oil-resistance is evaluated by checking the presence/absence of oil stain into the treated paper.
The case where no stain is observed is taken as "Good"; and the case where stain is observed is taken as "Poor".

### Oil-repellency (static contact angle) test

A test sample for the oil-repellency was prepared by spin coating a chloroform solution of 1.0% in solid content concentration of a heterocyclic ring compound on a glass substrate having a cellophane film pasted thereon, and annealing the resultant at 70°C for 10 min. As a solvent or a dispersion medium, chloroform was used. The static contact angel was measured. The static contact angle was obtained by dropping 2 µL of hexadecane (HD) on the resultant coating film, and measuring the contact angle at 1 s after the liquid dropping.

### Water-repellency test

The chloroform solution was coated on a thin paper and dried at room temperature to thereby fabricate a treated paper; thereafter, a water droplet was dropped and the case where the water droplet did not infiltrate for 30 min or longer was taken as acceptable.

### Example 1

### N², N⁴, N⁶-Trioctadecyl-1,3,5-triazine-2,4,6-triamine

On the above compound (Example 5 of JP No. 2006-342110 A), the KIT test, the corn oil-resistance test and the liquid-repellency test were carried out. The results are shown in Table 1.

### Example 2

### N², N⁶-Dioctadecanyl-2,6-pyridinedicarboxamide

4.90 g of stearylamine was dissolved in 26.0 mL of dehydrated chloroform, and thereafter cooled in an ice bath. After 10 min, 2.15 mL of triethylamine, and 1.5 g of pyridine-2,6-dicarboxylic dichloride were added in order, and stirred overnight. The next day, the reaction solution was filtered and the filtrate was concentrated, and the residue was stirred for 10 min in methanol. After 10 min, a solid was filtered and dried to thereby obtain 3.19 g of the above compound.
¹H NMR (CDCl₃, 400 MHz) δ: 0.87 (t, 6 H), 1.24-1.68 (m, 64 H), 3.47-3.52 (m, 2 H), 7.64 (m, 2 H), 8.01(t, 1 H), 8.33 (d, 2 H).

On the above compound, the KIT test, the corn oil-resistance test and the liquid-repellency test were carried out. The results are shown in Table 1.

### Example 3

### N²,N⁴,N⁶-trioctadecanyl-1,3,5-triazine-2,4,6-tricarboxamide

4.44 g of stearylamine was dissolved in 15.0 mL of dehydrated tetrahydrofuran, and thereafter, 1.3 g of triethyl-1,3,5-triazine-2,4,6-tricarboxylate was added, and stirred overnight in an oil bath at a temperature of 52°C. The next day, the resultant was cooled to room temperature and thereafter filtered and dried to thereby obtain 3.18 g of the above compound.
¹H NMR (CDCl₃, 400 MHz) δ: 0.87 (t, 9 H), 1.24-1.68 (m, 96 H), 3.10-3.58 (m, 6 H).

On the above compound, the KIT test, the corn oil-resistance test and the liquid-repellency test were carried out. The results are shown in Table 1.

**[Table 1]**

| | Oil-resistance | | Oil-repellency |
|---|---|---|---|
| | KIT (number) | Corn oil | Static contact angle (°) |
| Example 1 | 4 | ○ | 56.7 |
| Example 2 | 4 | ○ | 47.5 |
| Example 3 | 3 | ○ | 53.2 |

According to the results of the water-repellency test, Examples 1, 2 and 3 were proved to be acceptable.

### Example 4

### 2,4,6-Trioctadecyloxy-1,3,5-triazine

12.84 g of octadecyl alcohol was dissolved in 160 mL of dehydrated tetrahydrofurn, and thereafter, 1.87 g of NaH was added, and stirred for 1 hour. After 1 hour, 2.5 g of cyanuric chloride was added, and stirred overnight in an oil bath at a temperature of 72°C. The next day, the resultant was cooled to room temperature, and made acidic with hydrochloric acid, and concentrated. The residue was cleaned with methanol and dichloromethane to thereby obtain 21.97 g of the above compound.
¹H NMR (CDCl₃, 400 MHz) δ: 0.87 (t, 9 H), 1.24-1.68 (m, 96 H), 4.30-4.44 (m, 6 H).

500 mg of the above compound and 50 mg of a polyoxyethylene oleyl ether were heated at 100°C to melt the mixture. Thereafter, hot water was added under stirring so that the concentration of the above compound became 1% by weight, and the resultant was crushed by a mechanical shear homogenizer to thereby obtain a 1-wt% water dispersion. The dispersion was, three times, coated with an applicator set at a gap of 0 mil and dried, on a thin paper of 52 g/m² in water-resistance (Cobb value), 45 g/m² in basis weight, and 0.60 g/m³ in density, and heated at 140°C for 10 min to thereby obtain a treated paper. As a result of carrying out the corn oil-resistance test on the treated paper, no oil stain was observed.

### Industrial Applicability

The modified material (modified heterocyclic ring compound) of the heterocyclic ring compound of the present disclosure can be used as a water- and oil-repellent agent, and further as a water-resistant agent, a water-repellent agent, an oil-repellent agent, a soil-resistant agent, a soil release agent, a release agent and a mold release agent. The modified heterocyclic ring compound is suitably used in applications requiring the oil-resistance, in particular, in food applications such as food packaging materials and food containers.

## Claims

1. A water- and oil-repellent agent, comprising a modified heterocyclic ring compound having a hydrocarbon group having 1 to 40 carbon atoms and a heterocyclic ring.

2. The water- and oil-repellent agent according to claim 1, wherein the heterocyclic ring is a nitrogen-containing heterocyclic ring.

3. The water- and oil-repellent agent according to claim 1 or 2, wherein the hydrocarbon group is at least one selected from an octyl group, a decyl group, an undecyl group, a lauryl group, a tridecyl group, a myristyl group, a palmityl group, a cetyl group, an isopalmityl group, a stearyl group and a behenyl group.

4. The water- and oil-repellent agent according to any one of claims 1 to 3, wherein the heterocyclic ring is at least one selected from the group consisting of pyrimidine, pyridazine, pyridine, diazine, triazine, oxazole, oxadiazole, isoxazole, imidazole and pyrazine.

5. The water- and oil-repellent agent according to any one of claims 1 to 4, wherein the modified heterocyclic ring compound is a compound represented by the formula:
A(-B-R)ₙ
wherein
A is an optionally substituted heterocyclic ring;
B is -NR²-, -O-, -C(=O)NR²-, -NR²C(=O)-, -C(=O)O-, -OC(=O)-, -NR²C(=O)NR²-, or -OC(=O)NR²-, wherein R² are identical or different and each a hydrogen atom or a hydrocarbon group having 1 to 10 carbon atoms;
R is a hydrocarbon group having 1 to 40 carbon atoms and
optionally having a substituent; and
n is a number of 1 to 5.

6. The water- and oil-repellent agent according to any one of claims 1 to 5, wherein the water- and oil-repellent agent exhibits a contact angle of n-hexadecane of 10° or larger.

7. The water- and oil-repellent agent according to any one of claims 1 to 6, wherein the modified heterocyclic ring compound has a melting point of 40°C or higher or exhibits no melting point.

8. The water- and oil-repellent agent according to any one of claims 1 to 7, wherein the water- and oil-repellent agent in a solution concentration of 14.8 mg/mL has a viscosity of 5 cP or higher and 100 cP or lower.

9. The water- and oil-repellent agent according to any one of claims 1 to 8, wherein the water- and oil-repellent agent is a water dispersion composition.

10. The water- and oil-repellent agent according to any one of claims 1 to 9, wherein the water- and oil-repellent agent is for paper.

11. A textile product, comprising the modified heterocyclic ring compound in the water- and oil-repellent agent according to any one of claims 1 to 10 adhered thereon.

12. A water- and oil-repellent paper, comprising the modified heterocyclic ring compound in the water- and oil-repellent agent according to any one of claims 1 to 10 adhered thereon.

13. The water- and oil-repellent paper according to claim 12, wherein the water- and oil-repellent paper is a food packaging material or a food container.

14. A glass, comprising the modified heterocyclic ring compound in the water- and oil-repellent agent according to any one of claims 1 to 10 adhered thereon.

15. A method of treating the water- and oil-repellent paper according to claim 12 or 13, comprising subjecting the paper to an external addition treatment or an internal addition treatment.

16. A water dispersion composition, comprising: a modified heterocyclic ring compound having a hydrocarbon group having 1 to 40 carbon atoms and a heterocyclic ring; and a surfactant.

17. A compound, represented by the formula:
A(-B-R)ₙ
wherein
A is a pyridine ring, a diazine ring, or a triazine ring;
B is -NR²-, -O-, -C(=O)NR²-, -NR²C(=O)-, -C(=O)O-, -OC(=O)-, -NR²C(=O)NR²-, or -OC(=O)NR²-, wherein R² are identical or different and each a hydrogen atom or a hydrocarbon group having 1 to 10 carbon atoms;
R is a hydrocarbon group having 1 to 40 carbon atoms and optionally having a substituent; and
n is a number of 1 to 5.
